# EUROPÄISCHE PATENTSCHRIFT

(11) **EP 1 372 670 B1**
(45) Veröffentlichungstag und Bekanntmachung des Hinweises auf die Patenterteilung: **25.05.2005**
(21) Anmeldenummer: 02719903.3
(22) Anmeldetag: 23.02.2002
(51) Int. Cl.: A61K 31/70, A61P 35/00, C07H 13/06, C07H 15/18

(54) **CALOPOROSID-DERIVATE, VERFAHREN ZU DEREN HERSTELLUNG UND DEREN VERWENDUNG**
CALOPOROSIDE DERIVATIVES, METHODS FOR THE PRODUCTION THEREOF AND THEIR USE
DERIVES DE CALOPOROSIDE, LEUR PROCEDE DE PRODUCTION ET LEUR UTILISATION

(30) Priorität: 09.03.2001 DE 10111682
(43) Veröffentlichungstag der Anmeldung: 02.01.2004
(73) Patentinhaber: Aventis Pharma Deutschland GmbH, 65929 Frankfurt am Main (DE)
(72) Erfinder: EDER, Claudia, 65719 Hofheim (DE); KURZ, Michael, 65719 Hofheim (DE); BRÖNSTRUP, Mark, 60316 Frankfurt (DE); TOTI, Luigi, 65239 Hochheim (DE)
(86) Internationale Anmeldenummer: PCT/EP2002/001916
(87) Internationale Veröffentlichungsnummer: WO 2002/072110

(56) Entgegenhaltungen:
- WEBER W ET AL: "Caloporoside, a new inhibitor of phospholipases C from Caloporus dichrous (Fr.) Ryv." THE JOURNAL OF ANTIBIOTICS. JAPAN NOV 1994, Bd. 47, Nr. 11, November 1994 (1994-11), Seiten 1188-1194, XP001088232 ISSN: 0021-8820
- CRICH D ET AL: "Convergent, Stereoselective Synthesis of the Caloporoside Disaccharide" TETRAHEDRON LETTERS, ELSEVIER SCIENCE PUBLISHERS, AMSTERDAM, NL, Bd. 39, Nr. 51, 17. Dezember 1998 (1998-12-17), Seiten 9339-9342, XP004144195 ISSN: 0040-4039
- FAENZA I ET AL: "A role for nuclear phospholipase Cbeta 1 in cell cycle control." THE JOURNAL OF BIOLOGICAL CHEMISTRY. UNITED STATES 29 SEP 2000, Bd. 275, Nr. 39, 29. September 2000 (2000-09-29), Seiten 30520-30524, XP001088079 ISSN: 0021-9258
- HAJD CH M ET AL: "Synthetic cyclin dependent kinase inhibitors. New generation of potent anti-cancer drugs." ADVANCES IN EXPERIMENTAL MEDICINE AND BIOLOGY. UNITED STATES 1999, Bd. 457, 1999, Seiten 341-353, XP002201689 ISSN: 0065-2598
- ORTEGA^A^B S ET AL: "Cyclin D-dependent kinases, INK4 inhibitors and cancer" BBA - REVIEWS ON CANCER, ELSEVIER SCIENCE BV, AMSTERDAM, NL, Bd. 1602, Nr. 1, 14. März 2002 (2002-03-14), Seiten 73-87, XP004349034 ISSN: 0304-419X

## Beschreibung

Die vorliegende Erfindung betrifft neue Wirkstoffe (Caloporosid- Derivate), die von dem Mikroorganismus Gloeoporus dichrous Bres. ST001714, DSM 13784, während der Fermentation gebildet werden, Verfahren zu deren Herstellung, deren Verwendung als Arzneimittel, Caloporosid- Derivate enthaltende Arzneimittel sowie den Mikroorganismus Gloeoporus dichrous Bres. ST001714, DSM 13784.

Das Caloporosid wurde erstmals 1994 als Phospholipase C Inhibitor beschrieben (W. Weber et al. J. Antibiotics, 47, 1188-1194). Im gleichen Jahr wurden zwei weitere, ähnliche Sekundärmetaboliten isoliert (R. Shan et al. Nat. Prod. Lett., 4, 171-178). Die erfindungsgemäßen Verbindungen der Formel I (unten) unterscheiden sich von den dort beschriebenen Substanzen in ihrer Struktur.

Krebs ist eine zumeist tödlich verlaufende Erkrankung von Mensch und Tier, die durch das unkontrollierte Wachstum von körpereigenen Zellen verursacht wird. Krebs ist die Bezeichnung für die Bildung von bösartigen Geschwülsten (Malignome), von Neoplasmen (Tumoren bzw. Karzinome) oder für die maligne Entartung sowie Reifungsstörung weißer Blutzellen (Leukämie, Blutkrebs). Krebs- oder Tumorzellen entstehen durch Umwandlung körpereigener Zellen. Die Bösartigkeit der Krebszelle drückt sich in der Autonomie des Wachstums aus, das heißt in ihrer Fähigkeit, ungehemmt und ohne Einordnung in den Bauplan der Organe und unter Gewebszerstörung infiltrierend zu wachsen. Ein sicheres Zeichen der Malignität ist die Bildung tumorfemer Absiedlungen (Metastasen) nach hämatogener oder lymphogener Ausbreitung von Tumorzellen. Krebs gehört zu den häufigsten Todesursachen des Menschen und deshalb besteht ein großer Bedarf an Methoden und Mitteln zur Heilung oder Behandlung von malignen Entartungen.

Die Möglichkeit einer Therapie maligner Tumoren umfaßt neben der - wenn möglich radikalen - operativen Entfernung des Tumors, die radiologische Therapie mit Röntgenstrahlen, α-, β-, γ-Strahlen, die Immuntherapie und die Chemotherapie. Die Immuntherapie ist derzeit nur in eingeschränktem Maß anwendbar. Unter der Chemotherapie von Tumoren versteht man die Gabe von Zellgiften (Zytostatika) zur Behandlung von Tumoren und von verbliebenen Tumorzellen nach lokaler chirurgischer Behandlung oder Bestrahlung. Diese Stoffe greifen spezifisch in bestimmte Vorgänge der Zellteilung ein, so daß Gewebe mit hohem Anteil an sich teilenden Zellen, wie das rasch wachsende Tumorgewebe, empfindlicher reagieren. Zum Einsatz kommen alkylierende Verbindungen, wie z. B. das Cyclophosphamid (The Merck Index, 12. Ed. Seite 463), Antimetabolite, wie das Methotrexat (The Merck Index, 12. Ed. Seite 1025), Alkaloide, wie das Vincristin (The Merck Index, 12. Ed. Seite 1704) und Antibiotika, wie das Daunomycin (The Merck Index, 12. Ed. Seite 479) sowie das Adriamycin (The Merck Index, 12. Ed. Seite 581-582). All diese Agenzien haben aber aufgrund von massiven Nebenwirkungen große Nachteile, so daß der Tod der Erkrankten nur hinausgezögert, nicht jedoch abgewendet wird. Zudem treten bei entarteten (Krebs-) Zellen Resistenzen gegen die angewandten Mittel auf. Die derzeitigen Medikamente wirken dann nicht mehr zytostatisch, wohl aber toxisch infolge der Nebenwirkungen. Zudem hat sich gezeigt, daß eine kombinierte bzw. sequentielle Anwendung von Zytostatika die Wirksamkeit eines einzelnen Zytostatikums (Monotherapie) übertrifft und es dadurch möglich ist, daß sich die erheblichen Nebeneffekte bei der Polychemotherapie nicht addieren. Aus all diesen Gründen werden neue Chemotherapeutika dringend benötigt und deshalb weltweit gesucht.

Cyclin-abhängige Kinasen (cyclin-dependent kinases = CDKs) spielen eine zentrale Rolle bei der Regulation des Zellzykluses. Sie katalysieren Phosphorylierungsreaktionen und setzen damit eine Reaktionskaskade in Gang, die im Zellzyklus einen Übergang von der G1-Phase (Wachstumsphase 1) in die S-Phase (Synthesephase) initiiert. Cyclin-abhängige Kinasen stellen daher ein gutes therapeutisches Target für die Behandlung von Krebs und anderen Erkrankungen mit einer pathologischen Störung der Zellproliferation dar. Niedermolekulare Inhibitoren, die den Zellzyklus regulieren und unkontrollierte Zellteilung verhindern, wären hilfreiche Arzneistoffe bei der Behandlung von Krebspatienten.

Es ist überraschend gefunden worden, daß der Mikroorganismus-Stamm Gloeoporus dichrous (Fr. : Fr.) Bres. ST 001714, DSM 13784, hoch wirksame neue Zytostatika zu bilden vermag, die cyclin-abhängige Kinasen in sehr geringen Konzentrationen hemmen.

Gegenstand der Erfindung sind demzufolge die von dem Stamm Gloeoporus dichrous (Fr. : Fr.) Bres. ST 001714, DSM 13784 gebildeten Wirkstoffe (Caloporosid- Derivate) sowie deren physiologisch verträglichen Salze, Ester und offensichtliche chemische Äquivalente.

Die Erfindung betrifft somit Verbindungen der allgemeinen Formel I 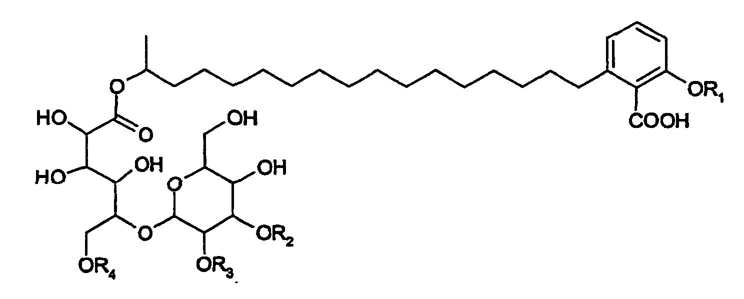 wobei bedeuten
R₁, R₂ und R₃, unabhängig voneinander, H oder ein Acylrest mit 2-10 C-Atomen, vorzugsweise 2 bis 6 Atomen, besonders bevorzugt 2 Atomen; und
R₄ H oder -C(O)(CH₂)ₙ COOH, worin n gleich 1 bis 7 ist, vorzugsweise 1 bis 3, besonders bevorzugt 1 oder 2;
mit der Ausnahme daß R₁, R₂, R₃ und R₄ nicht alle H bedeuten;
sowie deren physiologisch verträglichen Salze.

Die Acylreste der Verbindungen der Formel I können geradkettig oder verzweigt, gesättigt oder einfach oder zweifach ungesättigt sein.

Unter einem Acylrest mit 2C-Atomen ist beispielsweise ein Acetylrest zu verstehen.

Beispiele für gesättigte, unverzweigte Acylreste sind ein Essigsäurerest (C=2), Propionsäurerest (C=3), Buttersäurerest (C=4), Valeriansäurerest (C=5), Capronsäurerest (C=6), Önanthsäurerest (C=7), Caprylsäurerest (C=8), Penagonsäurerest (C=9) und Caprinsäurerest (C=10).

Beispiele für einfach ungesättigte, unverzweigte Acylreste sind ein Acrylsäurerest (C=3), Crotonsäurerest (C=4) oder ein Vinylessigsäurerest (C=4).

Ein Beispiel für zweifach ungesättigte, unverzweigte Acylrest ist ein Sorbinsäurerest (C=6).

Die Caloporoside sind schwach wirksame Antibiotika, die aus einer Salicylsäure und einem Disaccharid bestehen. Die beiden Struktureinheiten sind über eine Alkylkette verbunden. Der Zuckerteil der Verbindung der Formel I kann ein Disaccharid sein, bestehend jeweils aus einer D-Pyranose einer Aldohexose (wie z.B. D-Glucopyranose oder D- Galaktopyranose) und der Onsäure einer Aldohexose (z.B. D-Gluconsäure). Bevorzugt ist der Zuckerteil D-Mannopyranosyl-D-mannonsäure die unsubstituiert oder substituiert ist durch R₂, R₃ und/oder R₄ wie oben definiert.

Die Erfindung betrifft des weiteren
a) eine Verbindung der Formel I wobei R₁ = Acetyl; R₂ = R₃ = R₄ = H (=Caloporosid B: Summenformel: C₃₈H₆₂O₁₆, MG 774,9) sowie deren physiologisch verträglichen Salze;
b) eine Verbindung der Formel I wobei R₁ = R₃ = Acetyl; R₂ = H; R₄ = Malonyl (=Caloporosid C: Summenformel: C₄₃H₆₆O₂₀, MG 902,99) sowie deren physiologisch verträglichen Salze;
c) eine Verbindung der Formel I wobei R₁ = R₂ = H; R₃ = Acetyl; R₄ = Malonyl (=Caloporosid D: Summenformel: C₄₁H₆₄O₁₉, MG 806,96) sowie deren physiologisch verträglichen Salze;
d) eine Verbindung der Formel I wobei R₁ = R₃ = H; R₂ = Acetyl; R₄ = Malonyl (=Caloporosid E: Summenformel: C₄₁H₆₄O₁₉, MG 806.96) sowie deren physiologisch verträglichen Salze;
e) eine Verbindung der Formel I wobei R₁ = R₂ = R₄ = H; R₃ = Acetyl (=Caloporosid F: Summenformel: C₃₈H₆₂O₁₆, MG 774.9) sowie deren physiologisch verträglichen Salze.

Chiralitätszentren in den Verbindungen der Formel I können, wenn nicht anders angegeben, in der R- oder in der S-Konfiguration vorliegen. Die Erfindung betrifft sowohl die optisch reinen Verbindungen als auch Stereoisomerengemische wie Enantiomerengemische und Diasteromerengemische.

Erfindungsgemäß sind die Verbindungen der Formel I erhältlich durch Fermentation von Gloeoporus dichrous (Fr. : Fr.) Bres. ST001714, DSM 13784, oder eines seiner Varianten oder Mutanten unter geeigneten Bedingungen in einem Kulturmedium, bis sich eines oder mehrere der Caloporosid- Derivate der Formel I im Kulturmedium anhäufen. Durch anschließende Isolierung der Verbindungen und gegebenenfalls Umwandlung in chemische Äquivalente, sowie deren physiologisch verträglichen Salze werden die Caloporosid-Derivate gewonnen.

Die Erfindung betrifft daher weiterhin ein Verfahren zur Herstellung einer Verbindung der Formel I, dadurch gekennzeichnet, daß der Mikroorganismus Gloeoporus dichrous (Fr. : Fr.) Bres. ST001714, DSM 13784 oder einer seiner Varianten oder Mutanten unter geeigneten Bedingungen in einem Kulturmedium fermentiert wird, bis sich eine oder mehrere der Zielverbindungen in dem Kulturmedium anhäufen und anschließend aus dem Kulturmedium isoliert und gegebenenfalls in chemische Äquivalente und /oder physiologisch verträgliche Salze umgewandelt werden.

Vorzugsweise wird der Stamm ST001714, DSM 13784, seine Mutanten und/oder Varianten in einer Nährlösung oder einem Festmedium (auch als Kulturmedium bezeichnet) mit Kohlenstoff- und Stickstoffquelle sowie den üblichen anorganischen Salzen fermentiert, bis sich die erfindungsgemäßen Verbindungen in dem Kulturmedium anhäufen, anschließend werden die Verbindungen aus dem Kulturmedium isoliert und gegebenenfalls in die einzelnen aktiven Komponenten aufgetrennt.

Das erfindungsgemäße Verfahren kann für die Fermentation im Labormaßstab (Milliliter- bis Literbereich) und für den industriellen Maßstab (Kubikmetermaßstab) eingesetzt werden.

Der Stamm Gloeoporus dichrous (Fr. : Fr.) Bres. ST 001714, wurde in einer Vorkultur vermehrt. Ein Isolat wurde bei der Deutschen Sammlung von Mikroorganismen und Zellkulturen GmbH, Mascheroder Weg 1B, 3300 Braunschweig, Deutschland, nach den Regeln des Budapester Vertrages am 14. Dezember 1999 unter der folgenden Nummer hinterlegt: DSM 13784.

Gloeoporus dichrous (Fr. : Fr.) Bres. ST001714, DSM 13784, besitzt ein weißes Mycel und purpurfarbige Sporen. Er kommt vorzugsweise auf Betula vor, kann aber auch andere Wirte zum Beispiel Alnus, Salix, Populus, Ulmus, Prunus befallen.

Anstelle des Stammes Gloeoporus dichrous (Fr. : Fr.) Bres. ST001714, DSM 13784, können auch dessen Mutanten und Varianten eingesetzt werden, die ein oder mehrere der erfindungsgemäßen Verbindungen synthetisieren. Solche Mutanten können in an sich bekannter Weise durch physikalische Mittel, beispielsweise Bestrahlung, wie mit Ultraviolett- oder Röntgenstrahlen, oder chemische Mutagene, wie beispielsweise Ethylmethansulfonat (EMS), 2-Hydroxy-4-methoxy-benzophenon (MOB) oder N-Methyl-N'-nitro-N-nitrosoguanidin (MNNG) erzeugt werden.

Das Screening nach Mutanten und Varianten, die ein oder mehrere der erfindungsgemäßen Verbindungen synthetisieren, erfolgt nach folgendem Schema:
- Lyophilisierung der Plattenkulturen;
- Extraktion des Lyophilisats mit einem organische Lösungsmittel;
- Extraktion der Verbindung aus dem Kulturfiltrat mit Festphasen
- Analytik mittels HPLC, DC oder durch Testen der biologischen Wirksamkeit.

Die im folgenden beschriebenen Fermentationsbedingungen gelten für Gloeoporus dichrous (Fr. : Fr.) Bres. ST001714, das hinterlegte Isolat DSM 13784 sowie Mutanten und Varianten von diesen.
In einer Nährlösung, die eine Kohlenstoffquelle und eine Stickstoffquelle sowie die üblichen anorganischen Salze enthält, produziert Gloeoporus dichrous (Fr. : Fr.) Bres. ST001714, DSM 13784, die Caloporosid- Derivate.

Als bevorzugte Kohlenstoffquellen für die Fermentation eignen sich assimilierbare Kohlenhydrate und Zuckeralkohole, wie Glukose, Laktose, Saccharose oder D-Mannit sowie kohlenhydrathaltige Naturprodukte, wie z.B. Malzextrakt. Als stickstoffhaltige Nährstoffe kommen in Betracht: Aminosäuren, Peptide und Proteine sowie deren Abbauprodukte, wie Casein, Peptone oder Tryptone, ferner Fleischextrakte, Hefeextrakte, gemahlene Samen, beispielsweise von Mais, Weizen, Bohnen, Soja oder der Baumwollpflanze, Destillationsrückstände der Alkoholherstellung, Fleischmehle oder Hefeextrakte, aber auch Ammoniumsalze und Nitrate, insbesondere aber auch synthetisch bzw. biosynthetisch gewonnene Peptide. An anorganischen Salzen kann die Nährlösung beispielsweise Chloride, Carbonate, Sulfate oder Phosphate der Alkali- oder Erdalkalimetalle, Eisen, Zink, Kobalt und Mangan enthalten.

Die Bildung der erfindungsgemäßen Verbindungen verläuft besonders gut in einer Nährlösung, die etwa 0,05 bis 5 %, bevorzugt 1 bis 2 % Malzextrakt, 0,05 bis 3 %, bevorzugt 0.05 bis 1 % Hefeextrakt und 0,2 bis 5 %, bevorzugt 0,5 bis 2 % Glucose, 0,5 bis 3 %, bevorzugt 0,5 bis 3 % Cellulose Pulver und Spuren von Ammoniumsulfat enthalten. Die Angaben in Prozent sind jeweils bezogen auf das Gewicht der gesamten Nährlösung.

In dieser Nährlösung bildet Gloeoporus dichrous (Fr. : Fr.) Bres *ST001714,* DSM 13784, ein Gemisch aus Caloporosid-Derivaten. Je nach Zusammensetzung der Nährlösung kann der mengenmäßige Anteil eines oder mehrerer der erfindungsgemäßen Caloporosid- Derivate variieren. Außerdem kann durch die Medienzusammensetzung die Synthese einzelner Caloporosid- Derivate gesteuert werden, so daß ein Caloporosid- Derivat gar nicht bzw. in einer Menge unterhalb der Nachweisgrenze vom Mikroorganismus hergestellt wird.

Die Kultivierung des Mikroorganismus erfolgt aerob, also beispielsweise submers unter Schütteln oder Rühren in Schüttelkolben oder Fermentem gegebenenfalls unter Einführen von Luft oder Sauerstoff, oder auf Festmedium. Sie kann in einem Temperaturbereich von etwa 18 bis 35°C, vorzugsweise bei etwa 20 bis 30°C, insbesondere bei 25 bis 30°C durchgeführt werden. Der pH-Bereich sollte zwischen 5 und 8 liegen, vorzugsweise zwischen 5,5 und 6,5. Man kultiviert den Mikroorganismus unter diesen Bedingungen im allgemeinen über einen Zeitraum von 24 bis 720 Stunden, vorzugsweise 288 bis 576 Stunden.

Vorteilhaft kultiviert man in mehreren Stufen, d. h. man stellt zunächst eine oder mehrere Vorkulturen in einem flüssigen Nährmedium her, die dann in das eigent-liche Produktionsmedium, die Hauptkultur, beispielsweise im Volumenverhältnis 1:10, überimpft werden. Die Vorkultur erhält man, z.B., indem man ein Mycel in eine Nährlösung überimpft und etwa 36 bis 120 Stunden, bevorzugt 48 bis 72 Stunden, wachsen läßt. Das Mycel kann beispielsweise erhalten werden, indem man den Stamm etwa 3 bis 40 Tage, bevorzugt 10 bis 30 Tage, auf einem festen oder flüssigen Nährboden, beispielsweise Malz-Hefe-Agar oder Kartoffel-Dextrose-Agar wachsen läßt.

Der Fermentationsverlauf kann anhand des pH-Wertes der Kulturen oder des Mycelvolumens sowie durch chromatographische Methoden, wie z.B. Hochleistungsflüssigchromatographie (HPLC), oder Ausprüfen der biologischen Aktivität überwacht werden.

Das im folgenden beschriebene Isolierungsverfahren dient zur Aufreinigung der erfindungsgemäßen Caloporosid- Derivate.

Die Isolierung bzw. Aufreinigung eines erfindungsgemäßen Caloporosid- Derivates aus dem Kulturmedium erfolgt nach bekannten Methoden unter Berücksichtigung der chemischen, physikalischen und biologischen Eigenschaften der Naturstoffe. Zum Testen der Konzentration der jeweiligen Caloporosid- Derivate im Kulturmedium oder in den einzelnen Isolierungsstufen kann die HPLC verwendet werden, wobei die Menge der gebildeten Substanz zweckmäßig mit einer Eichlösung verglichen wird.

Zur Isolierung der erfindungsgemäßen Verbindungen werden die Kulturbrühe oder die Kultur samt Festmedium lyophilisiert, anschließend werden die Caloporosid- Derivate vom Lyophilisat mit einem gegebenenfalls mit Wasser mischbaren organischen Lösungsmittel extrahiert. Die organische Lösungsmitteiphase enthält die erfindungsgemäßen Naturstoffe, sie wird gegebenenfalls im Vakuum konzentriert und weiter aufgereinigt.

Die weitere Aufreinigung eines oder mehrerer erfindungsgemäßen Verbindungen erfolgt durch Chromatographie an geeigneten Materialien, vorzugsweise z.B. an Molekularsieben, an Kieselgel, Aluminiumoxid, an Ionenaustauschern oder an Adsorberharzen bzw. an Umkehr-Phasen (reversed phase, RP). Mit Hilfe dieser Chromatographie werden die Caloporosid- Derivate getrennt. Die Chromatographie der Caloporosid- Derivate erfolgt mit gepufferten wässrigen Lösungen oder Gemischen von wässrigen und organischen Lösungen.

Unter Gemischen von wässrigen oder organischen Lösungen versteht man alle mit Wasser mischbaren organischen Lösemittel, vorzugsweise Methanol, Propanol und Acetonitril, in einer Konzentration von 5 bis 80 % Lösemittel, vorzugsweise 20 bis 50 % Lösemittel oder auch alle gepufferten wässrigen Lösungen, die mit organischen Lösemitteln mischbar sind. Die zu verwendenden Puffer sind die gleichen wie oben angegeben.

Die Trennung der Caloporosid- Derivate aufgrund ihrer unterschiedlichen Polarität erfolgt mit Hilfe der Reversed Phase Chromatographie, beispielsweise an MCI® (Adsorberharz von Mitsubishi, Japan) oder Amberlite XAD® (TOSOHAAS), an weiteren hydrophoben Materialien, wie zum Beispiel an RP-8- oder RP-18-Phasen. Außerdem kann die Trennung mit Hilfe der Normalphasen-Chromatographie, zum Beispiel an Kieselgel, Aluminiumoxyd und ähnlichen erfolgen.

Die Chromatographie der Caloporosid- Derivate erfolgt mit gepufferten oder angesäuerten wäßrigen Lösungen oder Gemischen von wäßrigen Lösungen mit Alkoholen oder anderen, mit Wasser mischbaren organischen Lösemitteln. Als organisches Lösemittel wird vorzugsweise Propanol und Acetonitril verwendet.

Unter gepufferten oder angesäuerten wäßrigen Lösungen versteht man z.B. Wasser, Phosphatpuffer, Ammoniumacetat, Citratpuffer in einer Konzentration von 0 bis 0,5 M sowie Ameisensäure, Essigsäure, Trifluoressigsäure oder allen handelsüblichen, dem Fachmann bekannten Säuren, vorzugsweise in einer Konzentration von 0 bis 1 %. Bei gepufferten wäßrigen Lösungen wird 0,1 % Ammoniumacetat besonders bevorzugt

Chromatographiert wird mit einem Gradienten, der mit 100 % Wasser beginnt und mit 100 % Lösemittel endet, vorzugsweise wird ein linearer Gradient von 20 bis 100 % Propanol oder Acetonitril gefahren.

Alternativ kann auch eine Gelchromatographie oder die Chromatographie an hydrophoben Phasen erfolgen.

Die Gelchromatographie wird an Polyacrylamid- oder Mischpolymergelen durchgeführt, wie z.B. Biogel-P 2® (Fa. Biorad) oder Fractogel TSK HW 40® (Fa. Merck, Deutschland oder Toso Haas, USA).

Die Reihenfolge der vorgenannten Chromatographien ist umkehrbar.

Die erfindungsgemäßen Verbindungen sind im festen Zustand und in Lösungen im pH-Bereich zwischen 3 und 8, insbesondere 5 und 7, stabil und lassen sich damit in übliche galenische Zubereitungen einarbeiten.

Eine oder mehrere Verbindungen der erfindungsgemäßen Verbindungen eignen sich aufgrund ihrer wertvollen pharmakologischen Eigenschaften zur Anwendung in der Human- oder Tiermedizin als Arzneimittel.

Die vorliegende Erfindung betrifft somit die Verwendung der Verbindung der Formel I oder ein physiologisch verträgliches Salz desselben zur Herstellung eines Zytostatikums zur Behandlung von Tumorerkrankungen.

Die vorliegende Erfindung betrifft weiterhin alle offensichtlichen chemischen Äquivalenten der erfindungsgemäßen Verbindungen der Formel I. Solche Äquivalente sind Verbindungen, die einen geringfügigen chemischen Unterschied aufweisen, also die gleiche Wirkung haben oder sich unter milden Bedingungen in die erfindungsgemäßen Verbindungen umwandeln. Zu den genannten Äquivalenten gehören z.B. auch Salze, Reduktionsprodukte, Ester, Ether, Acetale oder Amide der erfindungegemäßen Verbindungen sowie Äquivalente die der Fachmann mit Standardmethoden herstellen kann, darüber hinaus alle optische Antipoden, Diastereomere und alle stereomere Formen.

Unter physiologisch verträglichen Salzen von Verbindungen der Formel I versteht man sowohl deren organische als auch anorganische Salze, wie sie in Remington's Phannaceutical Sciences (17. Auflage, Seite 1418 (1985)) beschrieben sind. Aufgrund der physikalischen und chemischen Stabilität und der Löslichkeit sind für saure Gruppen unter anderem Natrium-, Kalium-, Calcium- und Ammoniumsalze bevorzugt; für basische Gruppen sind unter anderem Salze der Salzsäure, Schwefelsäure, Phosphorsäure oder von Carbonsäuren oder Sulfonsäuren, wie z.B. Essigsäure, Zitronensäure, Benzoesäure, Maleinsäure, Fumarsäure, Weinsäure und p-Toluolsulfonsäure bevorzugt.

Ester, Ether und Acetale können nach literaturbeschriebenen Methoden hergestellt werden, z.B. in Advanced Organic Synthesis, 4^{th} Edition, J. March, John Wiley & Sons, 1992 oder Protective Groups in Organic Synthesis 3^{rd} Edition, T. W. Greene & P. G. M. Wuts, John Wiley & Sons, 1999.

Die Carboxylgruppe kann beispielsweise mit LiAlH₄ zum Alkohol reduziert werden.

Von Verbindungen der Formel I kann zunächst der Glykosidteil mittels alkalischer Hydrolyse abgespalten werden (W. Weber et al. J. Antibiotics, 47, 1188-1194). Anschließend können durch Glykosylierung (z.B. Königs-Knorr-Reaktion) beliebige Zuckerreste eingeführt werden. Entsprechende Methoden sind literaturbeschrieben z.B. in Carbohydrate Chemistry, J. F. Kennedy, Oxford University Press, 1988.

Der Wirkmechanismus der Caloporosid- Derivate ist unbekannt, jedoch konnte ein signifikanter Effekt nachgewiesen werden.

Zum Nachweis der Inhibitoren von CDKs bedient man sich eines Testes, bei dem die Phosphorylierungsrate eines spezifischen Peptidsubstrates durch die cyclin-abhängigen Kinasen gemessen wird. Die cyclin-abhängigen Kinasen werden durch Bindung an das jeweilige Cyclin aktiviert. [γ-P]-Phosphat wird von [γ-P]-ATP durch das Enzym auf das Peptidsubstrat übertragen. Der Test wird in 96-well Mikrotiterplatten durchgeführt: Gemessen wird die Radioaktivität des auf das Substrat übertragenen [γ-P]-Phosphats.

IC₅₀ Werte für die Caloporosid- Derivate sind in der Tabelle 1 angegeben; es ist die Konzentration, die CDK-4 zu 50 % inaktiviert.

| | |
|---|---|
| Caloporoside B | 1,5 µM |
| Caloporoside C | 3,1 µM |
| Caloporoside D | 1,8 µM |
| Caloporoside E | 1,8 µM |
| Caloporoside F | 1,5 µM |

Des weiteren betrifft die vorliegende Erfindung Arzneimittel mit einem Gehalt an mindestens einer erfindungsgemäßen Verbindung.

Ein oder mehrere Verbindungen der erfindungsgemäßen Caloporosid- Derivate können grundsätzlich als solche in Substanz verabreicht werden. Bevorzugt ist die Verwendung in Mischung mit geeigneten Hilfsstoffen oder Trägermaterial. Als Trägermaterial können bei Arzneimitteln die üblichen und pharmakologisch verträglichen Trägermaterialien und/oder Hilfsstoffe verwendet werden.

Die erfindungsgemäßen Arzneimittel werden im allgemeinen oral oder parenteral verabreicht, aber auch eine rektale Anwendung ist prinzipiell möglich. Geeignete feste oder flüssige galenische Zubereitungsformen sind beispielsweise Granulate, Pulver, Tabletten, Dragees, (Mikro-)Kapseln, Zäpfchen, Sirupe, Emulsionen, Suspensionen, Aerosole, Tropfen oder injizierbare Lösungen in Ampullenform sowie Präparate mit protrahierter Wirkstoff-Freigabe, bei deren Herstellung üblicherweise Trägerstoffe und Zusätze und/oder Hilfsmittel wie Spreng-, Binde-, Überzugs-, Quellungs-, Gleit- oder Schmiermittel, Geschmacksstoffe, Süßungsmittel oder Lösungsvermittler Verwendung finden. Als häufig verwendete Träger- oder Hilfsstoffe seien z.B. Magnesiumcarbonat, Titandioxid, Laktose, Mannit und andere Zucker, Talkum, Milcheiweiß, Gelatine, Stärke, Vitamine, Cellulose und ihre Derivate, tierische oder pflanzliche Öle, Polyethylenglykole und Lösungsmittel, wie etwa steriles Wasser, Alkohole, Glycerin und mehrwertige Alkohole genannt.

Gegebenenfalls können die Dosierungseinheiten für die orale Verabreichung mikroverkapselt werden, um die Abgabe zu verzögern oder über einen längeren Zeitraum auszudehnen, wie beispielsweise durch Überziehen oder Einbetten des Wirkstoffs in Teilchenform in geeignete Polymere, Wachse oder dergleichen.

Vorzugsweise werden die pharmazeutischen Präparate in Dosierungseinheiten hergestellt und verabreicht, wobei jede Einheit als aktiven Bestandteil eine bestimmte Dosis einer oder mehrerer Verbindungen der erfindungsgemäßen Caloporosid-Derivate enthält. Bei festen Dosierungseinheiten wie Tabletten, Kapseln und Suppositorien kann diese Dosis bis zu etwa 500 mg, bevorzugt jedoch etwa 0,1 bis 200 mg, und bei Injektionslösungen in Ampullenform bis zu etwa 200 mg, vorzugsweise aber etwa 0,5 bis 100 mg, pro Tag betragen.

Die zu verabreichende Tagesdosis ist abhängig vom Körpergewicht, Alter, Geschlecht und Zustand des Säugers. Unter Umständen können jedoch auch höhere oder niedrigere Tagesdosen angebracht sein. Die Verabreichung der Tagesdosis kann sowohl durch Einmalgabe in Form einer einzelnen Dosierungseinheit oder aber in mehreren kleineren Dosierungseinheiten als auch durch Mehrfachgabe unterteilter Dosen in bestimmten Intervallen erfolgen.

Die erfindungsgemäßen Arzneimittel werden dadurch hergestellt, daß man ein oder mehrere der erfindungsgemäßen Verbindungen mit üblichen Träger- sowie gegebenenfalls Zusatz- und/oder Hilfstoffe in die bzw. eine geeignete Darreichungsform bringt

In den sich anschließenden Beispielen wird die Erfindung weiter erläutert. Prozentangaben beziehen sich auf das Gewicht. Mischungsverhältnisse bei Flüssigkeiten beziehen sich auf das Volumen, wenn keine anderen Angaben gemacht wurden.

### Beispiele

### Beispiel 1: Herstellung einer Glycerinkultur von Gloeoporus dichrous (Fr. : Fr.) Bres. ST001714, DSM 13784

100 ml Nährlösung (Malzextrakt 2,0%, Hefeextrakt 0,2 %, Glukose 1,0 % (NH₄)₂ HPO₄ 0,05 %, pH 6,0) in einem sterilen 300 ml Erlenmeyerkolben werden mit dem Stamm Gloeoporus dichrous (Fr. : Fr.) Bres. *ST001714,* DSM 13784 und 7 Tage bei 25° C und 140 UpM auf einer rotierenden Schüttelmaschine inkubiert. 1,5 ml dieser Kultur werden anschließend mit 2,5 ml 80 %igem Glycerin verdünnt und bei -135°C gelagert.

### Beispiel 2: Herstellung einer Vorkultur im Erlenmeyerkolben von Gloeoporus dichrous (Fr. : Fr.) Bres. ST001714, DSM 13784.

30 ml Nährlösung (Malzextrakt 2,0%, Hefeextrakt 0,2 %, Glukose 1,0 % (NH₄)₂HPO₄ 0,05 %, pH 6,0) in einem sterilen 100 ml Erlenmeyerkolben werden mit dem Stamm Gloeoporus dichrous (Fr. : Fr.) Bres. *ST001714*, DSM 13784, beimpft und 4 Tage bei 25° C und 140 UpM auf einer rotierenden Schüttelmaschine inkubiert. Mit jeweils 2 ml dieser Vorkultur werden anschließend die Platten bei der Herstellung der Hauptkulturen beimpft.

### Beispiel 3: Herstellung einer Hauptkultur von Gloeoporus dichrous (Fr. : Fr.) Bres. ST001714, DSM 13784 auf Festmedium-Platten.

Sterile 25x25 cm Platten (Fa. Nunc) werden mit 200 ml der folgende Nährlösung 20 g/l Malzextrakt, 2 g/l Hefeextrakt, 10 g/l Glukose, und 0.5 g/l (NH₄)₂ HPO₄ pH 6.0 gegossen. Diese Platten wurden jeweils mit 2 ml einer Vorkultur beimpft. Die maximale Produktion einer oder mehrerer der erfindungsgemäßen Verbindungen ist nach ca. 480 Stunden erreicht.

### Beispiel 4: Herstellung der Caloporosid- Derivate

50 Stück 25x25 cm Platten wurden hergestellt und mit der Vorkultur beimpft:
Nährmedium:
   20 g/l Malzextrakt
   2 g/l Hefeextrakt
   10 g/l Glukose
   0.5 g/l (NH₄)₂ HPO₄
   pH 6 (vor der Sterilisation)
Inkubationszeit: 480 Stunden
Inkubationstemperatur: 25° C

### Beispiel 5: Isolierung des Caloporosid-Gemisches aus den Plattenkulturen von Gloeoporus dichrous (Fr. : Fr.) Bres. ST001714, DSM 13784.

Nach Beendigung der Fermentation von Gloeoporus dichrous (Fr. : Fr.) Bres. ST001714, DSM 13784, werden die Plattenkulturen, gewonnen nach Beispiel 3, lyophilisiert und das Lyophilisat mit 5 Liter Methanol extrahiert. Die wirkstoffhaltige, methanolische Lösung wird durch Filtration vom Rückstand befreit und im Vakuum konzentriert. Das Konzentrat wird mit Wasser verdünnt und auf eine vorbereitete, 1,0 Liter MCI GEL, CHP20P-Säule aufgetragen. Eluiert wird mit einem Gradienten von Wasser nach 100% Acetonitril. Der Säulendurchfluß (25 ml pro Minute) wird fraktioniert aufgefangen (je 25 ml) und die Caloporosid- Derivate enthaltenden Fraktionen (von 40% bis 100 % Acetonitril) zusammengefaßt. Konzentrieren im Vakuum und anschließende Lyophilisierung ergeben 8,5 g eines gelbbraunen Pulvers.

### Beispiel 6: Vortrennung der Caloporosid- Derivate durch RP18-Chromatographie.

0,5 g des nach Beispiel 4 gewonnenen Produktes werden auf eine Nucleosil® 100-7 C18 HD Säule (Größe: 40 mm x 250 mm) aufgetragen. Eluiert wird mit einem Gradienten von 20 % Acetonitril (+ Wasser mit 0,1 % Ammoniumacetat-Zusatz) nach 100% Acetonitril mit einer Flußrate von 35 ml pro Minute. Der Säulenausfluß wird fraktioniert (35 ml) aufgefangen. Hauptsächlich in den Fraktionen 39 bis 68 befinden sich die Caloporosid- Derivate. Sie werden zusammengefaßt, im Vakuum vom Lösungsmittel befreit und anschließend lyophilisiert. Dabei wurden Caloporosid B (22,4 mg) und F (10,5 mg) bereits > 95 % rein erhalten. Caloporosid C (Fraktion 41; 43,4 mg), D (Fraktion 43-45; 76,2 mg) und E (Fraktion 43-45; 76,2 mg) wurden zu ca. 70% rein erhalten und daher noch weiter chromatographisch aufgereinigt.

### Beispiel 7: Reinigung der Caloporoside C, D und E.

20 mg des nach Beispiel 5 isolierten und angereicherten Caloporosids C werden auf eine LUNA® 5µ C18(2) Säule (Größe: 10 mm x 250 mm) aufgetragen und mit einem Gradienten von 25 bis 35 % Acetonitril in 0,1 % Ammoniumacetat/Wasser chromatographiert. Der Durchfluß des Elutionsmittels beträgt 6,5 ml pro Minute, die Fraktionsgröße 6,5 ml. In den Fraktionen 35 bis 42 befindet sich das Caloporosid C. Die Lyophilisierung der genannten Fraktionen ergibt > 95 % reines Caloporosid C (7,5 mg)
25 mg des nach Beispiel 5 isolierten und angereicherten Gemisches aus Caloporosid D und E werden auf eine LUNA® 5µ C18(2) Säule (Größe: 10 mm x 250 mm) aufgetragen und mit einem Gradienten von 30 bis 40 % Acetonitril in 0,1 % Ammoniumacetat/Wasser chromatographiert. Der Durchfluß des Elutionsmittels beträgt 6,5 ml pro Minute, die Fraktionsgröße 6,5 ml. In den Fraktionen 18 und 19 befindet sich das Caloporosid D, in den Fraktionen 20 bis 21 Caloporosid E. Die Lyophilisierung der genannten Fraktionen ergibt > 95 % reines Caloporosid D (7,0 mg) und Caloporosid E (6,0 mg).

Die physikalisch-chemischen sowie spektroskopischen Eigenschaften der erfindungsgemäßen Substanzen lassen sich wie folgt zusammenfassen:

### Caloporosid B:.

Summenformel: C₃₈H₆₂O₁₆
Molekulargewicht: 774,9
UV-Maxima: 208,244,310
¹H- und ¹³C-NMR: siehe Tabelle 2

Das hochauflösende FAB Massenspektrum zeigt ein intensives MH⁺ bei m/z 775.4120 Da, in guter Übereinstimmung mit der berechneten Masse (für C₃₈H₆₃O₁₆, mono-isotopisch) von 775.4116 Da.

### Caloporosid C:

Summenformel: C₄₃H₆₆O₂₀
Molekulargewicht: 902,99
UV-Maxima: 208, 244, 310
¹H- und ¹³C-NMR: siehe Tabelle 3

Das hochauflösende FAB Massenspektrum zeigt ein intensives M+H⁺ bei m/z 903.4264 Da, in guter Übereinstimmung mit der berechneten Masse (für C₃₆H₇₁O₂₅, mono-isotopisch) von 903.4284 Da.

### Caloporosid D:

Summenformel: C₄₁H₆₄O₁₉
Molekulargewicht: 860.96
UV-Maxima: 208, 244, 310
¹H- und ¹³C-NMR: siehe Tabelle 4

Das hochauflösende FAB Massenspektrum zeigt ein intensives M+Na⁺ bei m/z 883.3942 Da, in guter Übereinstimmung mit der berechneten Masse (für C₄₁H₆₄O₁₉Na, mono-isotopisch) von 883.3939 Da.

### Caloporosid E:

Summenformel: C₄₁H₆₄O₁₉
Molekulargewicht: 860.96
UV-Maxima: 208, 244, 310
¹H- und ¹³C-NMR: siehe Tabelle 4

Das hochauflösende FAB Massenspektrum zeigt ein intensives M+Na⁺ bei m/z 883.3942 Da, in guter Übereinstimmung mit der berechneten Masse (für C₄₁H₆₄O₁₉Na, mono-isotopisch) von 883.3939 Da.

### Caloporosid F:

Summenformel: C₃₈H₆₂O₁₆
Molekulargewicht: 774.9
UV-Maxima: 208, 244, 310
¹H- und ¹³C-NMR: siehe Tabelle 5

Das hochauflösende FAB Massenspektrum zeigt ein intensives M+H⁺ bei m/z 775.4128 Da, in guter Übereinstimmung mit der berechneten Masse (für C₃₈H₆₃O₁₆, mono-isotopisch) von 775.4116 Da.

**Tabelle 2:**

| ¹H- und ¹³C-chemische Verschiebungen von Caloporosid B in Methanol-d₄ und DMSO-d₆ bei 300K | | | | |
|---|---|---|---|---|
| | DMSO | MeOD | DMSO | MeOD |
| 1 | - | - | 171.24 | ^{a)} |
| 2 | - | - | ^{a)} | ^{a)} |
| 3 | - | - | 162.22 | 163.57 |
| 4 | 6.68 | 6.77 | 115.28 | 116.81 |
| 5 | 7.17 | 7.25 | ~131.6 | b |
| 6 | 6.57 | 6.67 | 121.02 | ~123.0 |
| 7 | - | - | 137.90 | 139.57 |
| 8 | ^{a)} | ^{a)} | ^{a)} | ^{a)} |
| 9 | ^{a)} | ^{a)} | ^{a)} | ^{a)} |
| 10-20 | 1.30-1.20 | 1.32-1.27 | 29.01-28.69 | 30.76-30.47 |
| 21 | 1.30 | 1.38/1.32 | 24.81 | 26.52 |
| 22 | 1.54/1.45 | 1.65/1.52 | 35.31 | 37.00 |
| 23 | 4.81 | 4.95 | 70.04 | 73.09 |
| 24 | 1.15 | 1.24 | 19.69 | 20.22 |
| 1' | - | - | 173.43 | 175.35 |
| 2' | 3.95 | 4.13 | 70.89 | 72.97 |
| 3' | 3.95 | 4.17 | 70.04 | 71.59 |
| 4' | 3.54 | 3.82 | 67.96 | 69.80 |
| 5' | 3.65 | 3.86 | 78.94 | 80.12 |
| 6' | 3.74/3.44 | 3.90/3.69 | 62.11 | 63.25 |
| 1" | 4.53 | 4.71 | 100.27 | 101.34 |
| 2" | 3.74 | 3.95 | 70.37 | 72.46 |
| 3" | 3.22 | 3.44 | 73.67 | 75.30 |
| 4" | 3.21 | 3.49 | 67.54 | 69.00 |
| 5" | 3.04 | 3.22 | 77.15 | 78.23 |
| 6" | 3.72/3.31 | 3.88/3.63 | 61.80 | 63.25 |
| Ac-C' | - | ^{a)} | - | ^{a)} |
| Ac-Me | ^{a)} | ^{a)} | ^{a)} | ^{a)} |

| | | | | |
|---|---|---|---|---|
| a) Für diese Protonen/Kohlenstoffatome wird in MeOD, bzw. DMSO kein Signal beobachtet (Aggregation). | | | | |

**Tabelle 3:**

| ¹H- und ¹³C-chemische Verschiebungen von Caloporosid C in Methanol-d₄ bei 300K | | |
|---|---|---|
| | ¹H | ¹³C |
| 1 | - | 175.90 |
| 2 | - | 116.73 |
| 3 | - | 163.67 |
| 3-Ac-Me | ^{a)} | ^{a)} |
| 3-Ac-C' | - | ^{a)} |
| 4 | 6.73 | 116.73 |
| 5 | 7.16 | ~ 132.6 ^{b)} |
| 6 | 6.55 | 123.53 |
| 7 | - | 139.51 |
| 8 | 2.49 | 43.06 |
| 9 | 1.53 | 24.75 |
| 10-20 | 1.36-1.26 | 30.78-30.62 |
| 21 | 1.38/1.33 | 26.54 |
| 22 | 1.65/1.52 | 37.02 |
| 23 | 4.95 | 73.04 |
| 24 | 1.24 | 20.22 |
| 1' | - | 175.17 |
| 2' | 4.17 | 72.82 |
| 3' | 4.08 | 71.60 |
| 4' | 3.71 | 69.79 |
| 5' | 4.07 | 76.38 |
| 6' | 4.60/4.14 | 66.43 |
| 7' | - | 170.55 |
| 8' | 3.27^{(c} | ~44.8 |
| 9' | - | 173.12 |
| 1" | 4.93 | 99.40 |
| 2" | 5.33 | 73.40 |
| 3" | 3.72 | 73.40 |
| 4" | 3.42 | 69.34 |
| 5" | 3.34 | 78.19 |
| 6" | 3.89/3.62 | 63.11 |
| 2"-Ac-Me | 2.11 | 21.17 |
| 2"-Ac-C' | - | 172.56 |

| | | |
|---|---|---|
| a) Für diese Kerne wird kein Signal beobachtet (starke Signalverbreiterung). | | |
| b) Signalverbreiterung | | |
| c) Das Signal für die Protonen in Position 8' wird nur in der frisch angesetzten Lösung beobachtet (schneller Austausch gegen Deuterium). | | |

**Tabelle 4:**

| ¹H- und ¹³C-chemische Verschiebungen von Caloporosid D und E in Methanol-d₄ bei 300K | | | | |
|---|---|---|---|---|
| | ¹H Caloporosid D | 13C Caloporosid D | ¹H Caloporosid E | ¹³C Caloporosid E |
| 1 | - | - | 176.28 | 176.28 |
| 2 | - | - | 120.37 | 120.37 |
| 3 | - | - | 162.12 | 162.12 |
| 4 | 6.61 | 6.61 | 114.91 | 114.91 |
| 5 | 7.06 | 7.06 | 131.53 | 131.53 |
| 6 | 6.58 | 6.58 | 122.23 | 122.23 |
| 7 | - | - | 147.22 | 147.22 |
| 8 | 3.04 | 3.04 | 36.31 | 36.31 |
| 9 | 1.55 | 1.55 | 33.35 | 33.35 |
| 10-20 | 1.36-1.25 | 1.36-1.25 | 31.07-30.61 | 31.07-30.61 |
| 21 | 1.38/1.32 | 1.38/1.32 | 26.53 | 26.53 |
| 22 | 1.65/1.53 | 1.65/1.53 | 37.01 | 37.01 |
| 23 | 4.95 | 4.95 | 73.13^{a)} | 73.07^{a)} |
| 24 | 1.24 | 1.24 | 20.23 | 20.23 |
| 1' | - | - | 175.18 | 175.40 |
| 2' | 4.17 | 4.15 | 72.80 | 72.80 |
| 3' | 4.08 | 4.15 | 71.61 | 71.53 |
| 4' | 3.71 | 3.85 | 69.78 | 69.27 |
| 5' | 4.07 | 4.08 | 76.40 | 77.07 |
| 6' | 4.58/4.14 | 4.59/4.24 | 66.35 | 65.43 |
| 7' | - | - | ∼170.4 | ∼170.4 |
| 8' | 3.28 | 3.28 | ∼45.0 | ∼45.0 |
| 9' | - | - | ∼173.0 | ∼173.0 |
| 1" | 4.93 | 4.84 | 99.40 | 100.20 |
| 2" | 5.33 | 4.03 | 73.41 | 70.27 |
| 3" | 3.72 | 4.78 | 73.39 | 77.44 |
| 4" | 3.42 | 3.71 | 69.32 | 66.35 |
| 5" | 3.34 | 3.38 | 78.35 | 78.00 |
| 6" | 3.91/3.62 | 3.89/3.65 | 63.09 | 62.96 |
| 2"/3"-Ac-Me | 2.11 | 2.00 | 21.18 | 20.98 |
| 2"/3"-Ac-C' | - | - | 172.59 | 172.36 |

| | | | | |
|---|---|---|---|---|
| a) Diese Signale können nicht eindeutig zugeordnet werden. | | | | |

**Tabelle 5:**

| ¹H- und ¹³C-chemische Verschiebungen von Caloporosid F in Methanol-d₄ bei 300K | | |
|---|---|---|
| | ¹H | ¹³C |
| 1 | - | 174.95 |
| 2 | - | 117.32 |
| 3 | - | 162.18 |
| 4 | 6.68 | 115.42 |
| 5 | 7.17 | 133.17 |
| 6 | 6.67 | 122.65 |
| 7 | - | 146.91 |
| 8 | 2.93 | 36.57 |
| 9 | 1.56 | 33.27 |
| 10-20 | 1.36-1.25 | 30.93-30.62 |
| 21 | 1.35 | 26.53 |
| 22 | 1.65/1.53 | 37.02 |
| 23 | 4.95 | 73.05 |
| 24 | 1.24 | 20.23 |
| 1' | - | 175.15 |
| 2' | 4.17 | 72.98 |
| 3' | 4.09 | 71.73 |
| 4' | 3.71 | 69.88 |
| 5' | 3.85 | 79.59 |
| 6' | 3.90/3.61 | 63.57 |
| 1" | 4.88 | 99.76 |
| 2" | 5.37 | 73.52 |
| 3" | 3.63 | 73.56 |
| 4" | 3.46 | 69.27 |
| 5" | 3.28 | 78.33 |
| 6" | 3.91/3.63 | 63.06 |
| Ac-C' | - | 173.03 |
| Ac-Me | 2.13 | 21.21 |

### Beispiel 8: Bioassay auf CDK-4 Inhibitoren

Für die Bestimmung des IC₅₀-Wertes werden von den erfindungsgemäßen Naturstoffen Stammlösungen in einer Konzentration von 10 mM hergestellt. 384-well FlashPlates werden mit 50 µl (50 µg/well) biotinyliertem Peptidsubstrat bei Raumtemperatur in 2 Stunden beschichtet und anschließend 3 x mit PBS Puffer gewaschen. Für die Reaktion werden 30 µl einer mit Puffer verdünnten Lösung der Caloporosid- Derivate und 20 µl einer vorgemischten ATP/CyclinD1/CDK4 Lösung (Endkonzentration: 1 µCi 33P-γ-ATP, 2 µM ATP und 1µg Enzymgemisch) auf die Platten pipettiert. Nach 2 Stunden Reaktionszeit bei 37° C werden die Platten 3 x mit je 80 µl Phosphorsäure 3% gewaschen und anschließend in einem MicroBeta Counter 30 Sekunden lang vermessen. Die Bestimmung der prozentualen Inhibierung erfolgt unter Zuhilfenahme mathematischer Gleichungen. Für die Bestimmung von IC₅₀-Werten werden 10 Konzentrationen einer frisch verdünnten DMSO-Lösung der erfindungsgemäßen Substanzen getestet.

## Patentansprüche

1. Verbindungen der Formel I 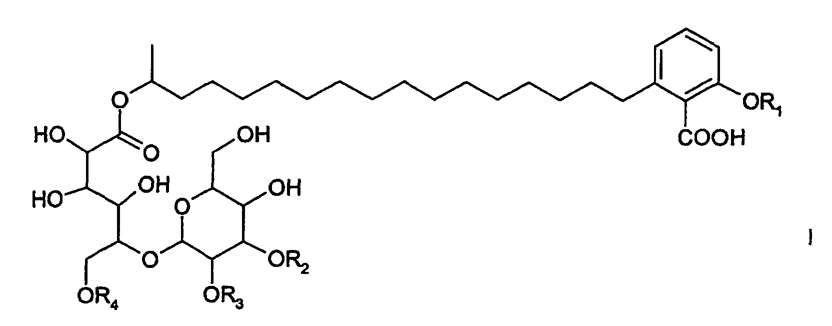 in der bedeuten:
R₁, R₂, R₃, unabhängig voneinander H oder Acylreste mit 1 bis 10 C-Atomen; und
R₄ H oder -C(O)(CH₂)ₙCOOH, worin n gleich 1 bis 7 ist;
mit der Ausnahme daß R₁, R₂, R₃ und R₄ nicht alle H bedeuten;
sowie deren physiologisch verträglichen Salze.

2. Verbindung der Formel I nach Anspruch 1 in der bedeuten:
R₁, R₂ und R₃, unabhängig voneinander H oder Acetyl; und
R₄ H oder Malonyl (n = 1);
sowie deren physiologische verträglichen Salze.

3. Verbindung der Formel I gemäß Anspruch 1 oder 2 in der bedeuten:
R₁ Acetyl; und
R₂, R₃ und R₄ H;
sowie deren physiologische verträglichen Salze.

4. Verbindung der Formel I gemäß Anspruch 1 oder 2 in der bedeuten:
R₁ und R₃ Acetyl;
R₂ H; und
R₄ Malonyl;
sowie deren physiologische verträglichen Salze.

5. Verbindung der Formel I gemäß Anspruch 1 oder 2 in der bedeuten:
R₁ und R₂ H;
R₃ Acetyl; und
R₄ Malonyl;
sowie deren physiologische verträglichen Salze.

6. Verbindung der Formel I gemäß Anspruch 1 oder 2 in der bedeuten:
R₁ und R₃ H;
R₂ Acetyl; und
R₄ Malonyl;
sowie deren physiologische verträglichen Salze.

7. Verbindung der Formel I gemäß Anspruch 1 oder 2 in der bedeuten:
R₁, R₂ und R₄ H; und
R₃ Acetyl:
sowie deren physiologische verträglichen Salze.

8. Verbindung der Formel I oder ein physiologisch verträgliches Salz davon gemäß einem oder mehreren der Ansprüche 1-7 herstellbar indem der Mikroorganismus Gloeoporus dichrous (Fr.: Fr.) Bres. ST001714, DSM 13784, bzw. einer seiner Varianten oder Mutanten unter geeigneten Bedingungen fermentiert wird, eine oder mehrere der Caloporosid- Derivate isoliert werden und diese gegebenenfalls in physiologisch verträglichen Salze überführt werden.

9. Verfahren zur Herstellung einer Verbindung der Formel I oder ein physiologisch verträgliches Salz davon gemäß einem oder mehreren der Ansprüche 1-7, **dadurch gekennzeichnet, daß** der Mikroorganismus Gloeoporus dichrous (Fr.: Fr.) Bres. ST001714, DSM 13784, bzw. einer seiner Varianten oder Mutanten unter geeigneten Bedingungen fermentiert wird, eine oder mehrere der Caloporosid- Derivate isoliert werden und diese gegebenenfalls in physiologisch verträgliche Salze überführt werden.

10. Verfahren gemäß Anspruch 9, **dadurch gekennzeichnet, daß** die Fermentation unter aeroben Bedingungen bei einer Temperatur zwischen 18 und 35°C und bei einem pH zwischen 5 und 8 durchgeführt wird.

11. Verbindung der Formel I oder ein physiologisch verträgliches Salz davon gemäß einem oder mehreren der Ansprüche 1-8 zur Anwendung als Arzneimittel.

12. Verbindung der Formel I, oder ein physiologisch verträgliches Salz davon gemäß einem oder mehreren der Ansprüche 1 - 8 zur Verwendung als CDK Inhibitor.

13. Verwendung von einer Verbindung der Formel I, oder ein physiologisch verträgliches Salz davon gemäß einem oder mehreren der Ansprüche 1-8 zur Herstellung von Arzneimitteln zur Behandlung von Krebs oder andereren Erkrankungen mit einer pathologischen Störung der Zellproliferation.

14. Arzneimittel mit einem Gehalt an mindestens einer Verbindung der Formel I, oder ein physiologisch verträgliches Salz davon gemäß einem oder mehreren der Ansprüche 1-8.

15. Verfahren zur Herstellung von Arzneimitteln gemäß Anspruch 14, **dadurch gekennzeichnet, daß** man mindestens eine Verbindung der Formel I, oder ein physiologisch verträgliches Salz davon gemäß einem oder mehreren der Ansprüche 1-8 den mit geeigneten Hilfs- und/oder Trägerstoffen in eine geeignete Darreichungsform bringt.

16. Mikroorganismus Gloeoporus dichrous (Fr.: Fr.) Bres. ST001714, DSM 13784.

## Claims

1. A compound of the formula I 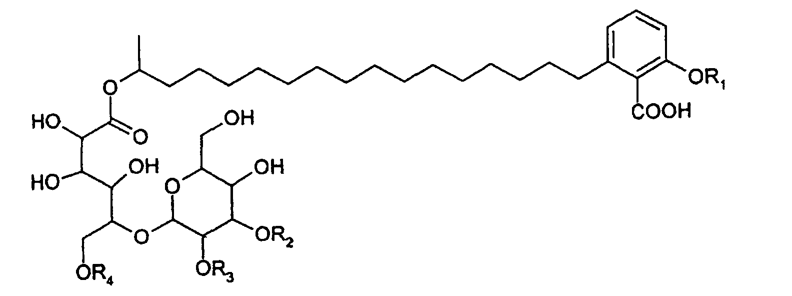 in which:
R₁, R₂, R₃ are, independently of one another, H or acyl radicals having 1 to 10 carbon atoms; and
R₄ is H or -C(O)(CH₂)ₙCOOH in which n is 1 to 7;
with the exception that R₁, R₂, R₃ and R₄ are not all H;
and the physiologically tolerated salts thereof.

2. A compound of the formula I as claimed in claim 1, in which
R₁, R₂, R₃ are, independently of one another, H or acetyl; and
R₄ is H or malonyl (n = 1);
and the physiologically tolerated salts thereof.

3. A compound of the formula I as claimed in claim 1 or 2, in which
R₁ is acetyl; and
R₂, R₃ and R₄ are H;
and the physiologically tolerated salts thereof.

4. A compound of the formula I as claimed in claim 1 or 2, in which
R₁ and R₃ are acetyl;
R₂ is H; and
R₄ is malonyl;
and the physiologically tolerated salts thereof.

5. A compound of the formula I as claimed in claim 1 or 2, in which
R₁ and R₂ are H;
R₃ is acetyl; and
R₄ is malonyl;
and the physiologically tolerated salts thereof.

6. A compound of the formula I as claimed in claim 1 or 2, in which
R₁ and R₃ are H;
R₂ is acetyl; and
R₄ is malonyl;
and the physiologically tolerated salts thereof.

7. A compound of the formula I as claimed in claim 1 or 2, in which
R₁, R₂ and R₄ are H; and
R₃ is acetyl;
and the physiologically tolerated salts thereof.

8. A compound of the formula I or a physiologically tolerated salt thereof as claimed in one or more of claims 1-7, which can be produced by fermentation of the microorganism Gloeoporus dichrous (Fr.:Fr.) Bres. ST001714, DSM 13784, or one of its variants or mutants under suitable conditions, isolation of one or more of the caloporoside derivatives and conversion thereof where appropriate into physiologically tolerated salts.

9. A process for the preparation of a compound of the formula I or a physiologically tolerated salt thereof as claimed in one or more of claims 1-7, which comprises fermentation of the microorganism Gloeoporus dichrous (Fr.:Fr.) Bres. ST001714, DSM 13784, or one of its variants or mutants under suitable conditions, isolation of one or more of the caloporoside derivatives and conversion thereof where appropriate into physiologically tolerated salts.

10. The process as claimed in claim 9, wherein the fermentation is carried out under aerobic conditions at a temperature between 18 and 35°C and at a pH between 5 and 8.

11. A compound of the formula I or a physiologically tolerated salt thereof as claimed in one or more of claims 1-8 for use as medicament.

12. A compound of the formula I or a physiologically tolerated salt thereof as claimed in one or more of claims 1-8 for use as CDK inhibitor.

13. The use of a compound of the formula I or a physiologically tolerated salt thereof as claimed in one or more of claims 1-8 for producing medicaments for the treatment of cancer or other disorders with a pathological disturbance of cell proliferation.

14. A medicament with a content of at least one compound of the formula I or a physiologically tolerated salt thereof as claimed in one or more of claims 1-8.

15. A process for producing medicaments as claimed in claim 14, which comprises converting at least one compound of the formula I or a physiologically tolerated salt thereof as claimed in one or more of claims 1-8 with suitable excipients and/or carriers into a suitable dosage form.

16. The microorganism Gloeoporus dichrous (Fr.:Fr.) Bres. ST001714, DSM 13784.

## Revendications

1. Composés de formule I 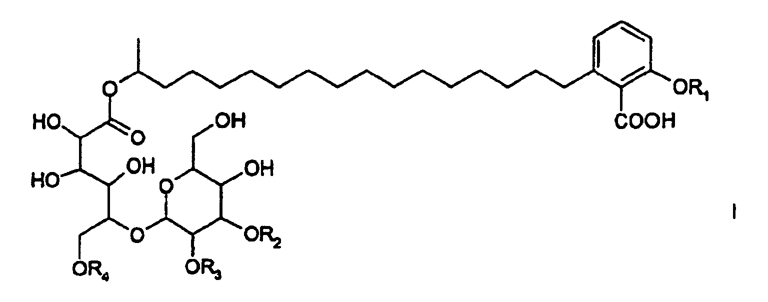 dans laquelle
R₁, R₂, R₃, signifient, indépendamment l'un de l'autre, H ou des radicaux acyle comprenant 1 à 10 atomes de carbone ; et
R₄ signifie H ou -C(O)(CH₂)ₙCOOH, où n vaut 1 à 7 ;
sauf que R₁, R₂, R₃ et R₄ ne signifient pas tous H
ainsi que leurs sels physiologiquement acceptables.

2. Composé de formule I selon la revendication 1, dans laquelle
R₁, R₂ et R₃ signifient, indépendamment l'un de l'autre H ou acétyle ; et
R₄ signifie H ou malonyle (n = 1) ;
ainsi que ses sels physiologiquement acceptables.

3. Composé de formule I selon la revendication 1 ou 2, dans laquelle
R₁ signifie acétyle ; et
R₂, R₃ et R₄ signifient H ;
ainsi que ses sels physiologiquement acceptables.

4. Composé de formule I selon la revendication 1 ou 2, dans laquelle :
R₁ et R₃ signifient acétyle ;
R₂ signifie H ; et
R₄ signifie malonyle ;
ainsi que ses sels physiologiquement acceptables.

5. Composé de formule I selon la revendication 1 ou 2, dans laquelle :
R₁ et R₂ signifient H ;
R₃ signifie acétyle ; et
R₄ signifie malonyle ;
ainsi que ses sels physiologiquement acceptables.

6. Composé de formule I selon la revendication 1 ou 2, dans laquelle :
R₁ et R₃ signifient H ;
R₂ signifie acétyle ; et
R₄ signifie malonyle ;
ainsi que ses sels physiologiquement acceptables.

7. Composé de formule I selon la revendication 1 ou 2, dans laquelle :
R₁, R₂ et R₄ signifient H ; et
R₃ signifie acétyle ;
ainsi que ses sels physiologiquement acceptables.

8. Composé de formule I ou un sel physiologiquement acceptable de celui-ci selon l'une ou plusieurs des revendications 1 à 7, pouvant être préparé en ce que le microorganisme Gloeoporus dichrous (Fr.: Fr.) Bres. ST001714, DSM 13784 ou une de ses variantes ou un de ses mutants est fermenté dans des conditions appropriées, un ou plusieurs des dérivés de caloporoside sont isolés et ceux-ci sont le cas échéant transformés en sels physiologiquement acceptables.

9. Procédé pour la préparation d'un composé de formule I ou d'un sel physiologiquement acceptable de celui-ci selon l'une ou plusieurs des revendications 1 à 7, **caractérisé en ce qu'**on fermente le microorganisme Gloeoporus dichrous (Fr.: Fr.) Bres. ST001714, DSM 13784, ou une de ses variantes ou un de ses mutants dans des conditions appropriées, on isole un ou plusieurs des dérivés de caloporoside et ceux-ci sont le cas échéant transformés en sels physiologiquement acceptables.

10. Procédé selon la revendication 9, **caractérisé en ce que** la fermentation est réalisée dans des conditions aérobies à une température entre 18 et 35°C et à un pH entre 5 et 8.

11. Composé de formule I ou un sel physiologiquement acceptable de celui-ci selon l'une ou plusieurs des revendications 1 à 8 destiné à une utilisation comme médicament.

12. Composé de formule I ou un sel physiologiquement acceptable de celui-ci selon l'une ou plusieurs des revendications 1 à 8 destiné à une utilisation comme inhibiteur des CDK.

13. Utilisation d'un composé de formule I ou d'un sel physiologiquement acceptable de celui-ci selon l'une ou plusieurs des revendications 1 à 8 pour la préparation de médicaments destinés au traitement du cancer ou d'autres maladies avec un trouble pathologique de la prolifération cellulaire.

14. Médicament présentant une teneur en au moins un composé de formule I, ou un sel physiologiquement acceptable de celui-ci selon l'une ou plusieurs des revendications 1 à 8.

15. Procédé pour la préparation de médicaments selon la revendication 14, **caractérisé en ce qu'**on amène au moins un composé de formule I ou un sel physiologiquement acceptable de celui-ci selon l'une ou plusieurs des revendications 1 à 8 avec les adjuvants et/ou supports appropriés en une forme d'administration appropriée.

16. Microorganisme Gloeoporus dichrous (Fr.: Fr.) Bres. ST001714, DSM 13784.
